(19) Europäisches Patentamt
European Patent Office
Office européen des brevets

(11) Veröffentlichungsnummer: **0 409 369 A2**

(12) **EUROPÄISCHE PATENTANMELDUNG**

(21) Anmeldenummer: **90250185.7**

(22) Anmeldetag: **19.07.90**

(51) Int. Cl.5: **C07D 239/60**, C07D 251/46, C07D 251/30, C07D 403/12, C07D 405/12, C07D 409/12, C07D 401/12, A01N 43/54, A01N 43/66

(30) Priorität: **19.07.89 DE 3924259**

(43) Veröffentlichungstag der Anmeldung:
**23.01.91 Patentblatt 91/04**

(84) Benannte Vertragsstaaten:
**AT BE CH DE DK ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Harde, Christoph, Dr.**
**Südwestkorso 61**
**D-1000 Berlin 41(DE)**
Erfinder: **Nordhoff, Erhard, Dr.**
**Markelstrasse 45**
**D-1000 Berlin 41(DE)**
Erfinder: **Krüger, Anita, Dr.**
**Schluchseestrasse 65**
**D-1000 Berlin 28(DE)**
Erfinder: **Krüger, Gabriele, Dr.**
**Bachstrasse 3**
**D-1000 Berlin 21(DE)**
Erfinder: **Tarara, Gerhard, Dr.**
**Sprengelstrasse 36**
**D-1000 Berlin 65(DE)**
Erfinder: **Wegner, Peter, Dr.**
**Münchener Strasse 3**
**D-1000 Berlin 28(DE)**
Erfinder: **Heinrich, Nikolaus, Dr.**
**Berliner Strasse 14 a**
**D-1000 Berlin 37(DE)**
Erfinder: **Rees, Richard, Dr.**
**Speerweg 8**
**D-1000 Berlin 28(DE)**
Erfinder: **Johann, Gerhard, Dr.**
**Hermsdorfer Damm 147**
**D-1000 Berlin 28(DE)**
Erfinder: **Kötter, Clemens, Dr.**
**Laurinsteig 26**
**D-1000 Berlin 28(DE)**

(54) **Substituierte Pyrimidinyloxy-(thio)- und Triazinyloxy(thio)-acrylsäurederivate, Verfahren zu ihrer Herstellung und ihre Verwendung als Mittel mit herbizider, fungizider und pflanzenwachstumsregulierender Wirkung.**

(57) Die Erfindung betrifft neue substituierte Pyrimidinyloxy(thio)- und Triazinyloxy(thio)acrylsäurederivate der allgemeinen Formel I

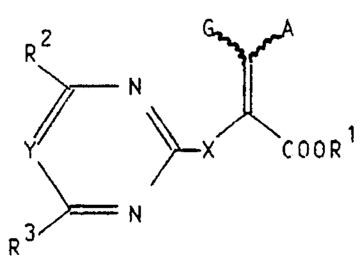

(I) ,

EP 0 409 369 A2

in der A, G, $R^1$, $R^2$, $R^3$, X und Y die in der Beschreibung genannten Bedeutungen haben, Verfahren zu ihrer Herstellung und ihre Verwendung als Mittel mit herbizider, fungizider und pflanzenwachstumsregulierender Wirkung.

# SUBSTITUIERTE PYRIMIDINYLOXY(THIO)- UND TRIAZINYLOXY(THIO)-. ACRYLSÄUREDERIVATE, VERFAHREN ZU IHRER HERSTELLUNG UND IHRE VERWENDUNG ALS MITTEL MIT HERBIZIDER, FUNGIZIDER UND PFLANZENWACHSTUMSREGULIERENDER WIRKUNG

Die Erfindung betrifft neue substituierte Pyrimidinyloxy(thio)- und Triazinyloxy(thio)acrylsäurederivate, Verfahren zu ihrer Herstellung und ihre Verwendung als Mittel mit herbizider, fungizider und pflanzenwachstumsregulierender Wirkung.

Es ist bereits bekannt, daß Pyrimidinderivate eine herbizide Wirkung besitzen (EP-Anmeldungen 0 223 406, 0 249 707, 0 249 708, 0 287 072 und 0 287 079). Häufig ist jedoch die Herbizidwirkung der bekannten Verbindungen nicht ausreichend, beziehungsweise es treten bei entsprechender Herbizidwirkung Selektivitätsprobleme in landwirtschaftlichen Hauptkulturen auf.

Aufgabe der vorliegenden Erfindung ist die Bereitstellung von neuen Verbindungen, die diese Nachteile nicht aufweisen und die in ihren biologischen Eigenschaften den bisher bekannten Verbindungen überlegen sind.

Es wurde nun gefunden, daß substituierte Pyrimidinyloxy(thio)- und Triazinyloxy(thio)acrylsäurederivate der allgemeinen Formel I

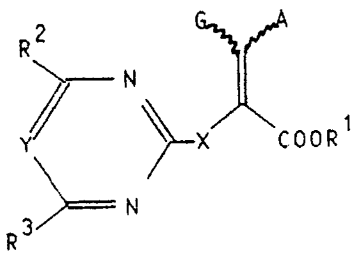

(I) ,

in der
A eine der Gruppen A - 1 bis A - 6 der allgemeinen Formeln

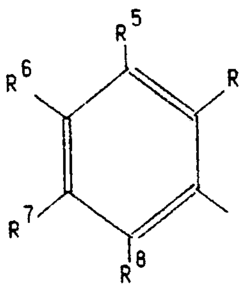

,

A - 1

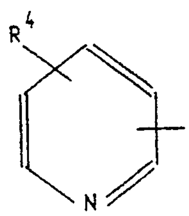

,

A - 2

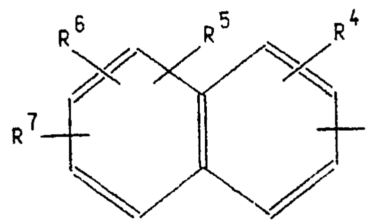

,

A - 3

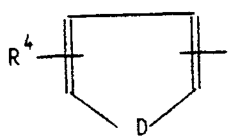

,

A - 4

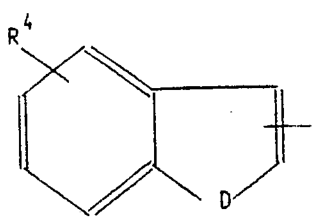

oder

A - 5

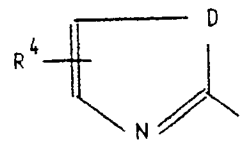

,

A - 6

einen $C_1$-$C_{10}$-Alkylrest, einen ein- oder mehrfach, gleich oder verschieden durch $C_1$-$C_4$-Alkoxy, $C_1$-$C_4$-

Alkylthio, Amino, $C_1$-$C_4$-Alkylamino, Di-$C_1$-$C_4$-alkylamino, Nitro, Trifluormethyl, Halogen oder Phenyl substituierten $C_1$-$C_{10}$-Alkylrest, einen $C_2$-$C_{10}$-Alkenylrest, einen ein- oder mehrfach, gleich oder verschieden durch $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_2$-$C_{10}$-Alkenylrest, einen $C_2$-$C_{10}$-Alkinylrest, einen ein- oder mehrfach, gleich oder verschieden durch $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_2$-$C_{10}$-Alkinylrest, einen $C_3$-$C_8$-Cycloalkylrest, einen ein- oder mehrfach, gleich oder verschieden durch $C_1$-$C_4$-Alkyl, $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_3$-$C_8$-Cycloalkylrest, einen $C_4$-$C_8$-Cycloalkenylrest oder einen ein- oder mehrfach, gleich oder verschieden durch $C_1$-$C_4$-Alkyl, $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_4$-$C_8$-Cycloalkenylrest,

D ein Sauerstoffatom, ein Schwefelatom oder eine Gruppe -$NR^9$-,

G ein Wasserstoffatom oder einen $C_1$-$C_6$-Alkylrest,

$R^1$ ein Wasserstoffatom, einen $C_1$-$C_4$-Alkylrest oder einen Benzylrest,

$R^2$ einen $C_1$-$C_4$-Alkylrest, einen $C_1$-$C_4$-Alkoxyrest, einen $C_1$-$C_4$-Alkylthiorest, einem $C_1$-$C_4$-Alkylaminorest, einen Di-$C_1$-$C_4$-alkylaminorest oder ein Halogenatom,

$R^3$ einen $C_1$-$C_4$-Alkylrest, einen $C_1$-$C_4$-Alkoxyrest, einen $C_1$-$C_4$-Alkylthiorest, einen $C_1$-$C_4$-Alkylaminorest, einen Di-$C_1$-$C_4$-alkylaminorest oder ein Halogenatom,

$R^4$ ein Wasserstoffatom, einen $C_1$-$C_4$-Alkylrest, einen $C_1$-$C_4$-Alkoxyrest, einen Aminorest, einen Di-$C_1$-$C_4$-alkylaminorest, einen Nitrorest, ein Halo genatom, einen Trifluormethylrest oder einen Phenylrest,

$R^5$ ein Wasserstoffatom, einen $C_1$-$C_4$-Alkylrest, einen $C_1$-$C_4$-Alkoxyrest, einen Aminorest, einen Di-$C_1$-$C_4$-alkylaminorest, einen Nitrorest, ein Halogenatom, einen Trifluormethylrest oder einen Phenylrest,

$R^6$ ein Wasserstoffatom, einen $C_1$-$C_4$-Alkylrest, einen $C_1$-$C_4$-Alkoxyrest, einen Aminorest, einen Di-$C_1$-$C_4$-alkylaminorest, einen Nitrorest, ein Halogenatom, einen Trifluormethylrest oder einen Phenylrest,

$R^7$ ein Wasserstoffatom, einen $C_1$-$C_4$-Alkylrest, einen $C_1$-$C_4$-Alkoxyrest, einen Aminorest, einen Di-$C_1$-$C_4$-alkylaminorest, einen Nitrorest, ein Halogenatom, einen Trifluormethylrest oder einen Phenylrest,

$R^8$ ein Wasserstoffatom, einen $C_1$-$C_4$-Alkylrest, einen $C_1$-$C_4$-Alkoxyrest, einen Aminorest, einen Di-$C_1$-$C_4$-alkylaminorest, einen Nitrorest, ein Halogenatom, einen Trifluormethylrest oder einen Phenylrest,

$R^9$ ein Wasserstoffatom, einen $C_1$-$C_4$-Alkylrest oder einen Phenylrest,

X ein Sauerstoffatom oder ein Schwefelatom und

Y eine Methingruppe oder ein Stickstoffatom

bedeuten, sowie deren Alkalimetallsalze, Erdalkalimetallsalze und organischen Ammoniumsalze und deren E- und Z-Isomere eine interessante herbizide, fungizide und pflanzenwachstumsregulierende Wirkung zeigen.

Die Bezeichnung Halogen umfaßt Fluor, Chlor, Brom und Jod. Unter der Bezeichnung Alkalimetall ist Lithium, Natrium und Kalium, unter der Bezeichnung Erdalkalimetall Calcium, Strontium und Barium zu verstehen.

Die erfindungsgemäßen Verbindungen der allgemeinen Formel I können zum Beispiel hergestellt werden, indem man

A) Verbindungen der allgemeinen Formel II

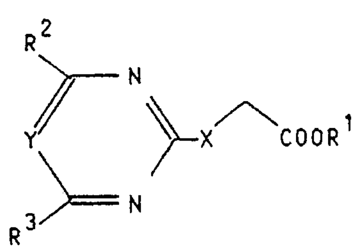

(II) ,

in der $R^1$, $R^2$, $R^3$, X und Y die unter der allgemeinen Formel I genannten Bedeutungen haben, mit Verbindungen der allgemeinen Formel III

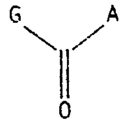

(III) ,

in der A und G die unter der allgemeinen Formel I genannten Bedeutungen haben, in einem geeigneten Lösungsmittel in Gegenwart einer geeigneten Base umsetzt, oder

B) Verbindungen der allgemeinen Formel IV

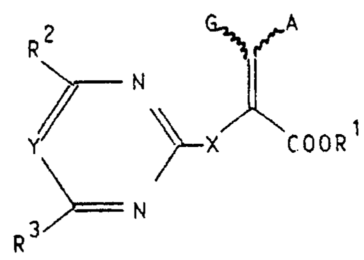

(IV) ,

in der A, G, $R^1$, $R^2$, $R^3$, X und Y die unter der allgemeinen Formel I angegebenen Bedeutungen haben, in einem geeigneten polaren Lösungsmittel mit einer Alkalimetallbase, Erdalkalimetallbase oder organischen Ammoniumbase zu Verbindungen der allgemeinen Formel V

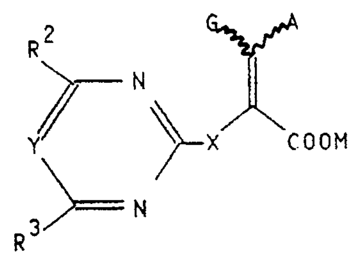

(V) ,

in der A, G, $R^2$, $R^3$, X und Y die unter der allgemeinen Formel I angegebenen Bedeutungen haben und M für ein Alkalimetallatom, ein Äquivalent eines Erdalkalimetallatoms oder eine organische Ammoniumgruppe steht, umsetzt, oder

C) Verbindungen der allgemeinen Formel V

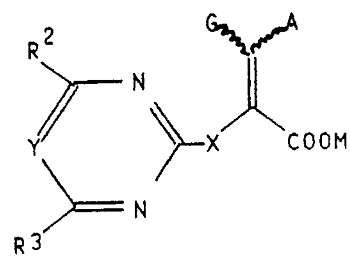

(V) ,

in der A, G, $R^2$, $R^3$, M, X und Y die unter der allgemeinen Formel V angegebenen Bedeutungen haben, mit einer geeigneten Säure in einem geeigneten Lösungsmittel zu Verbindungen der allgemeinen Formel VI

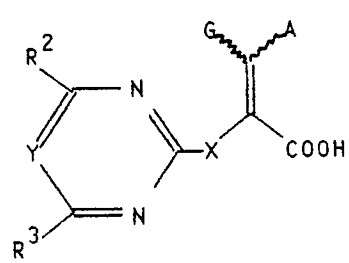

(VI) ,

in der A, G, $R^2$, $R^3$, X und Y die unter der allgemeinen Formel I angegebenen Bedeutungen haben, umsetzt, oder

D) Verbindungen der allgemeinen Formel IV

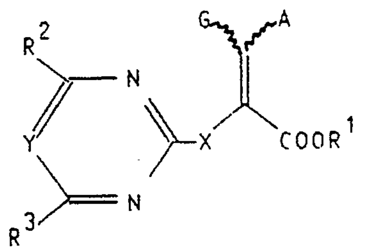

(IV) ,

in der A, G, $R^2$, $R^3$, X und Y die unter allgemeinen Formel I angegebenen Bedeutungen haben und $R^1$ für $C_1$-$C_4$-Alkyl oder Benzyl steht, mit einer geeigneten Säure in einem geeigneten Lösungsmittel zu einer Verbindung der allgemeinen Formel VI

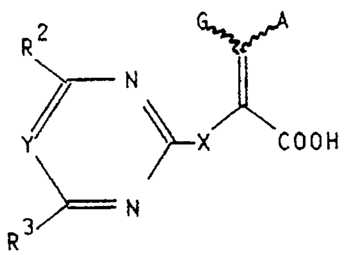

(VI) ,

in der A, G, $R^2$, $R^3$, X und Y die unter der allgemeinen Formel I angegebenen Bedeutungen haben, umsetzt.

Die einzelnen Verfahrensvarianten werden vorzugsweise in Gegenwart eines Verdünnungsmittels durchgeführt. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide und Sulfone, wie zum Beispiel Dimethylsulfoxid und Sulfolan, und Basen, wie zum Beispiel Pyridin.

Die Gegenwart eines Reaktionskatalysators kann von Vorteil sein. Als Katalysatoren sind Kaliumjodid und Oniumverbindungen geeignet, wie quaternäre Ammonium-, Phosphonium- und Arsoniumverbindungen sowie Sulfoniumverbindungen. Ebenfalls geeignet sind Polyglycolether, insbesondere cyclische, wie zum Beispiel 18-Krone-6, und tertiäre Amine, wie zum Beispiel Tributylamin. Bevorzugte Verbindungen sind quaternäre Ammoniumverbindungen, wie zum Beispiel Benzyltriethylammoniumchlorid und Tetrabutylammoniumbromid.

Die Reaktionen lassen sich bei dem Druck der Umgebung durchführen, wenngleich sie auch bei erhöhten beziehungsweise vermindertem Druck durchgeführt werden können.

Für die Verfahrensvariante A) können als Lösungsmittels bevorzugt aprotische Solventien, wie zum Beispiel Benzol, Toluol, Xylol, Tetrahydrofuran, Diethylether, Hexan, Dimethylformamid oder Dimethylsulfoxid verwendet werden.

Als Basen könnnen zum Beispiel Natriumhydrid, Kaliumtertiärbutylat oder Lithiumdiisopropylamid verwendet werden.

Die Reaktionstemperatur liegt zwischen -78°C und der Siedetemperatur des jeweiligen Lösungsmittels beziehungsweise Lösungsmittelgemisches.

Die Reaktionszeit beträgt 5 Minuten bis 48 Stunden, vorzugsweise 0,5 bis 24 Stunden.

Die Verbindungen der allgemeinen Formel II sind in der Literatur beschrieben, oder können nach literaturanalogen Verfahren hergestellt werden (Khim.-Farm. Zh. 16 (8), 931-4 [1982]; Ukr. Khim. Zh. (Russ.Ed.) 49 (11), 1205-8 [1983]; Fizol. Akt. Veshchestva 18 , 75-9 [1986]; USSR-PS 791746).

Für die Verfahrensvarianten B), C) und D) können als Lösungsmittel vorzugsweise Alkohole, wie zum Beispiel Methanol, Ethanol, Propanol oder Isopropanol, Ketone, wie zum Beispiel Aceton oder Methylethylketon, Dimethylformamid oder Dimethylsulfoxid, Wasser oder Lösungsmittel/Wasser-Gemische verwendet werden.

Als Alkalimetallbasen, Erdalkalimetallbasen oder organische Ammoniumbasen können zum Beispiel Carbonate, wie Natriumcarbonat, Kaliumcarbonat oder Calciumcarbonat, Metallhydroxide, wie Natriumhydroxid, Kaliumhydroxid, Bariumhydroxid oder Strontiumhydroxid und als organische Ammoniumbasen Ammoniak, Alkylamine (primäre Amine), Dialkylamine (sekundäre Amine) oder Trialkylamine (tertiäres Amine) verwendet werden.

Als Säuren kommen zum Beispiel Salzsäure oder Schwefelsäure in Frage.

Die Reaktionstemperatur liegt zwischen Raumtemperatur und der Siedetemperatur des jeweiligen Lösungsmittels. Die Reaktionszeit beträgt 0,5 bis 48 Stunden.

Falls $R^1$ in der allgemeinen Formel IV eine Benzylgruppe bedeutet, kann eine Verbindung der allgemeinen Formel VI auch durch katalytische Reduktion (Hydrierung) erhalten werden.

Die nach den oben genannten Verfahren hergestellten erfindungsgemäßen Verbindungen können nach den üblichen Verfahren aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion.

Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel farb- und geruchlose Flüssigkeiten sowie Kristalle dar, die löslich in Wasser, wenig löslich in aliphatischen Kohlenwasserstoffen, wie Petrolether, Hexan, Pentan und Cyclohexan, gut löslich in halogenierten Kohlenwasserstoffen, wie Chloroform, Methylenchlorid und Tetrachlorkohlenstoff, aromatischen Kohlenwasserstoffen, wie Benzol, Toluol und Xylol, Ethern, wie Diethylether, Tetrahydrofuran und Dioxan, Carbonsäurenitrilen, wie Acetonitril, Alkoholen, wie Methanol und Ethanol, Carbonsäureamiden, wie Dimethylformamid, und Sulfoxiden, wie Dimethylsulfoxid, sind.

Die erfindungsgemäßen Verbindungen zeigen eine gute herbizide Wirkung bei breitblättrigen Unkräutern und Gräsern. Ein selektiver Einsatz der erfindungsgemäßen Wirkstoffe ist in verschiedenen Kulturen möglich, zum Beispiel in Raps, Rüben, Sojabohnen, Baumwolle, Reis, Gerste, Weizen und anderen Getreidearten. Dabei sind einzelne Wirkstoffe als Selektivherbizide in Rüben, Baumwolle, Soja und Getreide besonders geeignet. Ebenso können die Verbindungen zur Unkrautbekämpfung in Dauerkulturen, wie zum Beispiel Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, und zur selektiven Unkrautbekämpfung in einjährigen Kulturen eingesetzt werden.

Die erfindungsgemäßen Verbindungen können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monochoria, Fimbristylis, Eleocharis, Ischaemum und Apera.

Die Aufwandmengen schwanken je Anwendungsart im Vor- und Nachauflauf in Grenzen zwischen 0,001 bis 5 kg/ha.

Die erfindungsgemäßen Wirkstoffe können auch als Defoliant, Desiccant und als Krautabtötungsmittel verwendet werden. Sie beeinflussen auch das Pflanzenwachstum und können deshalb zur Wachstumsbeeinflussung von Kulturpflanzen eingesetzt werden. Einige dieser Wirkstoffe zeigen auch eine fungizide Wirkung.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden. Beispielsweise eignen sich als herbizid wirksame Mischungspartner diejenigen Wirkstoffe, die in Weed Abstracts, Vol. 38, No. 3, 1989, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe, wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozen flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

1.) 25 Gewichtsprozent Wirkstoff
60 Gewichtsprozent Kaolin
10 Gewichtsprozent Kieselsäure
5 Gewichtsprozent einer Mischung aus dem Calciumsalz der Ligninsulfonsäure und dem Natriumsalz des N-Methyl-N-oleyl-taurins

2.) 40 Gewichtsprozent Wirkstoff
25 Gewichtsprozent Tonmineralien
25 Gewichtsprozent Kieselsäure
10 Gewichtsprozent einer Mischung aus dem Calciumsalz der Ligninsulfonsäure und Alkylphenylpolyglycolethern

B) **Paste**

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Gewichtsprozent Wasser

C) **Emulsionskonzentrat**

25 Gewichtsprozent Wirkstoff
15 Gewichtsprozent Cyclohexanon
55 Gewichtsprozent Xylol

5 Gewichtsprozent einer Mischung aus dem Calciumsalz der Dodecylbenzolsulfonsäure und Nonylphenylpolyoxyethylen

Die nachstehenden Beispiele erläutern die Herstellung der erfindungsgemäßen Verbindungen:

Beispiel 1

**2-(4,6-Dimethoxy-2-pyrimidinylthio)-3-phenylacrylsäure-methylester**

2,8 g (28,7 mmol) Diisopropylamin werden in 80 ml Tetrahydrofuran unter Stickstoff vorgelegt und mit 11,5 ml (28,7 mmol) 2,5 M n-Butyllithium in Hexan, bei -78 bis -50°C versetzt. Nach 10 minütigem Rühren gibt man langsam 7 g (28,7 mmol) 2-(4,6-Dimethoxy-2-pyrimidinylthio)-essigsäuremethylester, gelöst in 80 ml Tetrahydrofuran, zu der Reaktionslösung und rührt nach beendeter Zugabe noch 20 Minuten nach. Danach tropft man langsam 2,8 g (28,7 mmol) Benzaldehyd in die Lösung. Unter Rühren läßt man die Reaktionslösung sich langsam auf Raumtemperatur erwärmen und rührt noch 16 Stunden nach. Der Ansatz wird mit 500 ml Wasser versetzt und gründlich mit Essigester extrahiert. Die über Magnesiumsulfat getrocknete Essigesterphase wird eingedampft und anschließend durch Mitteldruckchromatographie mit Hexan/Essigester als Elutionsmittel gereinigt.

Ausbeute: 6,1 g = 63,9 % der Theorie

$n_D^{20}$ : 1,5926

Herstellung des Ausgangsmaterials

**2-(4,6-Dimethoxy-2-pyrimidinylthio)-essigsäure-methylester**

25 g (230 mmol) Thioglycolsäuremethylester werden in 250 ml Dimethylformamid vorgelegt und mit 16,3 g (115 mmol) Kaliumcarbonat versetzt. Nach 20 minütigem Rühren bei Raumtemperatur setzt man 50 g (230 mmol) 4,6-Dimethoxy-2-methylsulfonylpyrimidin zu und erwärmt für drei Stunden auf 90°C. Anschließend wird das Reaktionsgemisch auf Wasser gegeben und mit Essigester extrahiert. Die organische Phase wird mit Wasser gewaschen und anschließend über Magnesiumsulfat getrocknet. Das Lösungsmittel wird abdestilliert und das so erhaltene Rohprodukt aus Diisopropylether umkristallisiert.

Ausbeute: 44,6 g = 79,4 % der Theorie

Fp.: 67-69°C

Beispiel 2

**2-(4,6-Dimethoxy-2-pyrimidinylthio)-3-phenylacrylsäure**

0,5 g (1,5 mmol) 2-(4,6-Dimethoxy-2-pyrimidinylthio)-3-phenylacrylsäure-methylester und 95 mg (1,7 mmol) Kaliumhydroxid werden in ein Gemisch aus 5 ml Wasser und 5 ml Ethanol gegeben und 8 Stunden auf 50°C erhitzt. Das Gemisch wird mit etwa 100 ml Wasser verdünnt und mit Essigester extrahiert. Die wässrige Phase wird mit 10%iger Salzsäure bis auf pH 2 angesäuert und mit Essigester extrahiert. Nach dem Trocknen der Essigesterphase über Magnesiumsulfat wird das Lösungsmittel in Vakuum abgedampft und der Rückstand aus Diisopropylether umkristallisiert.

Ausbeute: 0,25 g = 52 % der Theorie

Fp.: 162-164°C

In analoger Weise wurden auch die folgenden Verbindungen der allgemeinen Formel I hergestellt:

| Beispiel Nr. | A | G | $R^1$ | $R^2$ | $R^3$ | X | Y | Physikalische Konstante |
|---|---|---|---|---|---|---|---|---|
| 3 | 2-Furyl | H | $CH_3$ | $OCH_3$ | $OCH_3$ | S | CH | Fp.: 106-108°C |
| 4 | 2-Furyl | H | H | $OCH_3$ | $OCH_3$ | S | CH | Fp.: 133-136°C |
| 5 | 2-Thienyl | H | $CH_3$ | $OCH_3$ | $OCH_3$ | S | CH | Fp.: 106-109°C |
| 6 | 2-Thienyl | H | H | $OCH_3$ | $OCH_3$ | S | CH | Fp.: 176°C (Zers.) |
| 7 | 2-Nitro-phenyl | H | $CH_3$ | $OCH_3$ | $OCH_3$ | S | CH | Fp.: 162-164°C |
| 8 | 2-Nitro-phenyl | H | H | $OCH_3$ | $OCH_3$ | S | CH | Fp.: 215°C (Zers.) |
| 9 | 1-Naphthyl | H | $CH_3$ | $OCH_3$ | $OCH_3$ | S | CH | Fp.: 95-96°C |
| 10 | 2-Naphthyl | H | $CH_3$ | $OCH_3$ | $OCH_3$ | S | CH | Fp.: 98-99°C |
| 11 | $CH_3CH=CH-$ | H | $CH_3$ | $OCH_3$ | $OCH_3$ | S | CH | Fp.: 40-42°C |
| 12 | $CH_3CH_2-$ | H | $CH_3$ | $OCH_3$ | $OCH_3$ | S | CH | $n_D^{20}$: 1,5431 |
| 13 | 2-Naphthyl | H | H | $OCH_3$ | $OCH_3$ | S | CH | Fp.: 159-161°C |
| 14 | 1-Naphthyl | H | H | $OCH_3$ | $OCH_3$ | S | CH | Fp.: 180-182°C |
| 15 | $H_2C=CH-$ | H | $CH_3$ | $OCH_3$ | $OCH_3$ | S | CH | Fp.: 40-43°C |
| 16 | $CH_3CH_2CH_2-$ | H | $CH_3$ | $OCH_3$ | $OCH_3$ | S | CH | $n_D^{20}$: 1,5381 |
| 17 | Phenyl | H | $CH_3$ | $OCH_3$ | $N(CH_3)_2$ | S | N | Fp.: 107-108°C |
| 18 | $(CH_3)_2CH-$ | H | $CH_3$ | $OCH_3$ | $OCH_3$ | S | CH | Fp.: 50-53°C |
| 19 | 2-Phenyl-ethyl | H | $CH_3$ | $OCH_3$ | $OCH_3$ | S | CH | $n_D^{20}$: 1,5711 |
| 20 | Phenyl | H | H | $OCH_3$ | $N(CH_3)_2$ | S | N | Fp.: 156-158°C |
| 21 | $CH_3CH_2CH_2-$ | H | H | $OCH_3$ | $OCH_3$ | S | CH | Fp.: 108-111°C |
| 22 | $(CH_3)_2CH-$ | H | H | $OCH_3$ | $OCH_3$ | S | CH | Fp.: 100-103°C |
| 23 | $CH_3-$ | H | $CH_3$ | $OCH_3$ | $OCH_3$ | O | CH | Fp.: 70-71°C |
| 24 | $CH_3-$ | H | H | $OCH_3$ | $OCH_3$ | O | CH | $R_f$: 0,26 (Essigester) |
| 25 | 3-Thienyl | H | $CH_3$ | $OCH_3$ | $OCH_3$ | S | CH | Fp.: 81°C |
| 26 | 3-Furyl | H | $CH_3$ | $OCH_3$ | $OCH_3$ | S | CH | Fp.: 115°C |
| 27 | N-Methyl-2-pyrrolyl | H | $CH_3$ | $OCH_3$ | $OCH_3$ | S | CH | Fp.: 109°C |

| Beispiel Nr. | A | G | $R^1$ | $R^2$ | $R^3$ | X | Y | Physikalische Konstante |
|---|---|---|---|---|---|---|---|---|
| 28 | 4-Nitro-phenyl | H | $CH_3$ | $OCH_3$ | $OCH_3$ | S | CH | Fp.: 148°C |
| 29 | 2-Pyrrolyl | H | $CH_3$ | $OCH_3$ | $OCH_3$ | S | CH | Fp.: 137°C |
| 30 | 2-Pyridyl | H | $CH_3$ | $OCH_3$ | $OCH_3$ | S | CH | Fp.: 110°C |
| 31 | 3-Pyridyl | H | $CH_3$ | $OCH_3$ | $OCH_3$ | S | CH | Fp.: 100°C |
| 32 | 2-Chlor phenyl | H | $CH_3$ | $OCH_3$ | $OCH_3$ | S | CH | Fp.: 100°C |
| 33 | 3-Chlor-phenyl | H | $CH_3$ | $OCH_3$ | $OCH_3$ | S | CH | Fp.: 110°C |
| 34 | 2-Methoxy-phenyl | H | $CH_3$ | $OCH_3$ | $OCH_3$ | S | CH | Fp.: 110°C |
| 35 | 2-Methyl-phenyl | H | $CH_3$ | $OCH_3$ | $OCH_3$ | S | CH | Fp.: 94°C |
| 36 | 3-Pyridyl | H | H | $OCH_3$ | $OCH_3$ | S | CH | Fp.: 190°C (Zers.) (x HCl) |
| 37 | 3-Methyl-phenyl | H | $CH_3$ | $OCH_3$ | $OCH_3$ | S | CH | Fp.: 90°C |
| 38 | 4-Fluor-phenyl | H | $CH_3$ | $OCH_3$ | $OCH_3$ | S | CH | $n_D^{20}$: 1,5618 |
| 39 | 3-Chlor-phenyl | H | H | $OCH_3$ | $OCH_3$ | S | CH | Fp.: 147°C |
| 40 | 4-Methyl-phenyl | H | $CH_3$ | $OCH_3$ | $OCH_3$ | S | CH | Fp.: 95°C |
| 41 | 2-Methyl-phenyl | H | H | $OCH_3$ | $OCH_3$ | S | CH | Fp.: 200-210°C (Zers.) |
| 42 | 3-Methyl-phenyl | H | H | $OCH_3$ | $OCH_3$ | S | CH | Fp.: 212°C (Zers.) |
| 43 | 2-Fluor-phenyl | H | $CH_3$ | $OCH_3$ | $OCH_3$ | S | CH | Fp.: 85°C |
| 44 | 2-Fluor-phenyl | H | H | $OCH_3$ | $OCH_3$ | S | CH | Fp.: 182°C (Zers.) |
| 45 | 4-Methoxy-phenyl | H | H | $OCH_3$ | $OCH_3$ | S | CH | Fp.: 195°C (Zers.) |

| Beispiel Nr. | A | G | $R^1$ | $R^2$ | $R^3$ | X | Y | Physikalische Konstante |
|---|---|---|---|---|---|---|---|---|
| 46 | 4-Fluor-phenyl | H | H | $OCH_3$ | $OCH_3$ | S | CH | Fp.: 188 °C (Zers.) |
| 47 | 4-Methoxy-phenyl | H | $CH_3$ | $OCH_3$ | $OCH_3$ | S | CH | Fp.: 80 °C |
| 48 | 4-Pyridyl | H | $CH_3$ | $OCH_3$ | $OCH_3$ | S | CH | Fp.: 81 °C |
| 49 | Cyclohexyl | H | $CH_3$ | $OCH_3$ | $OCH_3$ | S | CH | Fp.: 76-81 °C |
| 50 | $(CH_3)_3C$- | H | $CH_3$ | $OCH_3$ | $OCH_3$ | S | CH | $n_D^{20}$: 1,5308 |
| 51 | 2-Phenyl-ethyl | H | H | $OCH_3$ | $OCH_3$ | S | CH | $R_f$: 0 (Essigester) |
| 52 | Phenyl | H | $CH_3$ | $CH_3$ | $CH_3$ | S | CH | $n_D^{20}$: 1,5978 |
| 53 | Phenyl | H | $CH_2CH_3$ | $OCH_3$ | $OCH_3$ | S | CH | $n_D^{20}$: 1,5974 |
| 54 | Phenyl | H | $CH_3$ | $OCH_3$ | $OCH_3$ | S | N | - |
| 55 | Phenyl | H | H | $OCH_3$ | $OCH_3$ | S | N | |
| 56 | Phenyl | H | $CH_3$ | $OCH_3$ | $OCH_3$ | O | CH | Fp.: 106-109 °C |
| 57 | Phenyl | H | H | $OCH_3$ | $OCH_3$ | O | CH | $n_D^{20}$: 1,5759 |
| 58 | Phenyl | H | $CH_3$ | $OCH_3$ | $OCH_3$ | O | N | |
| 59 | Phenyl | H | H | $OCH_3$ | $OCH_3$ | O | N | |
| 60 | Phenyl | H | $CH_2CH_3$ | $OCH_3$ | $OCH_3$ | O | CH | $n_D^{20}$: 1,5638 |
| 61 | Phenyl | H | $CH_2CH_3$ | $OCH_3$ | $OCH_3$ | O | N | $n_D^{20}$: 1,5535 |

Die folgenden Beispiele erläutern die Anwendungsmöglichkeiten der erfindungsgemäßen Verbindungen:

## Beispiel A

Im Gewächshaus wurden die aufgeführten Pflanzenspezies nach dem Auflaufen mit den aufgeführten Verbindungen in einer Aufwandmenge von 0,1 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigte zwei Wochen nach der Behandlung die erfindungsgemäße Verbindung eine hohe Kulturpflanzenselektivität in Weizen und Mais bei ausgezeichneter Wirkung gegen das Unkraut. Das Vergleichsmittel zeigte nicht die gleichhohe Wirksamkeit.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
TRZAX = Triticum aestivum
ZEAMX = Zea mays
GALAP = Galium aparine
SEBEX = Sesbania exaltata
SOLSS = Solanum sp.

| Erfindungsgemäße Verbindung | TRZAX | ZEAMX | GALAP | SEBEX | SOLSS |
|---|---|---|---|---|---|
| Beispiel 1 | 0 | 0 | 3 | 3 | 3 |
| Unbehandelt | 0 | 0 | 0 | 0 | 0 |
| Vergleichsmittel | | | | | |
| 2-(4-Isopropyl-4-methyl-5-oxo-2-imidazolin-2-yl)-4(5)-methylbenzoesäuremethylester | 0 | 0 | 1 | 1 | 0 |

**Beispiel B**

Im Gewächshaus wurden die aufgeführten Pflanzenspezies vor dem Auflaufen mit den aufgeführten Verbindungen in einer Aufwandmenge von 0,1 kg Wirkstoff/ha behandelt. Die Verbindungen wurden zu diesem Zweck als Emulsionen oder Suspensionen mit 500 Litern Wasser/ha gleichmäßig über den Boden versprüht. Hier zeigten drei Wochen nach der Behandlung die erfindungsgemäßen Verbindungen eine hohe Kulturpflanzenselektivität in Weizen und Mais bei ausgezeichneter Wirkung gegen das Unkraut. Das Vergleichsmittel zeigte nicht die gleichhohe Wirksamkeit.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
TRZAX = Triticum aestivum
VERPE = Veronica persica
VIOSS = Viola sp.

| Erfindungsgemaße Verbindungen | TRZAX | VERPE | VIOSS |
|---|---|---|---|
| Beispiel 13 | 0 | 3 | 3 |
| Beispiel 25 | 0 | 3 | - |
| Unbehandelt | 0 | 0 | 0 |
| Vergleichsmittel | | | |
| 2-(4-Isopropyl-4-methyl-5-oxo-2-imidazolin-2-yl)-4(5)-methylbenzoesäuremethylester | 0 | 1 | 2 |

## Ansprüche

1. Substituierte Pyrimidinyloxy(thio)- und Triazinyloxy(thio)acrylsäurederivate der allgemeinen Formel I

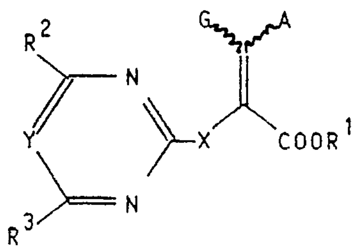

(I) ,

in der
A eine der Gruppen A - 1 bis A - 6 der allgemeinen Formeln

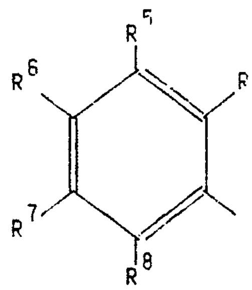 ,

A - 1

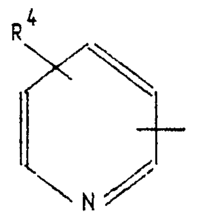 ,

A - 2

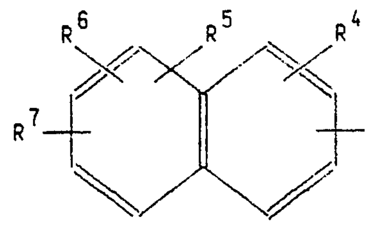 ,

A - 3

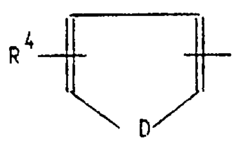 ,

A - 4

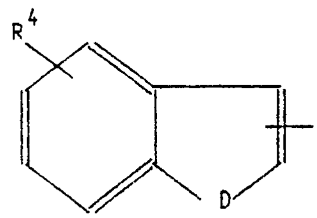

A - 5

oder

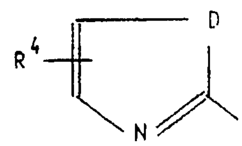 ,

A - 6

einen $C_1$-$C_{10}$-Alkylrest, einen ein- oder mehrfach, gleich oder verschieden durch $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_1$-$C_{10}$-Alkylrest, einen $C_2$-$C_{10}$-Alkenylrest, einen ein- oder mehrfach, gleich oder verschieden durch $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_2$-$C_{10}$-Alkenylrest, einen $C_2$-$C_{10}$-Alkinylrest, einen ein- oder mehrfach, gleich oder verschieden durch $C_1$-$C_4$-Alkoxy, $C_1$- $C_4$-Alkylthio, Amino, $C_1$-$C_4$-Alkylamino, Di-$C_1$-$C_4$-alkylamino, Nitro, Trifluormethyl, Halogen oder Phenyl substituierten $C_2$-$C_{10}$-Alkinylrest, einen $C_3$-$C_8$-Cycloalkylrest, einen ein- oder mehrfach, gleich oder verschieden durch $C_1$-$C_4$-Alkyl, $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_3$-$C_8$-Cycloalkylrest, einen $C_4$-$C_8$-Cycloalkenylrest oder einen ein- oder mehrfach, gleich oder verschieden durch $C_1$-$C_4$-Alkyl, $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_4$-$C_8$-Cycloalkenylrest,

D ein Sauerstoffatom, ein Schwefelatom oder eine Gruppe -$NR^9$-,

G ein Wasserstoffatom oder einen $C_1$-$C_6$-Alkylrest,

$R^1$ ein Wasserstoffatom, einen $C_1$-$C_4$-Alkylrest oder einen Benzylrest,

$R^2$ einen $C_1$-$C_4$-Alkylrest, einen $C_1$-$C_4$-Alkoxyrest, einen $C_1$-$C_4$-Alkylthiorest, einen $C_1$-$C_4$-Alkylaminorest, einen Di-$C_1$-$C_4$-alkylaminorest oder ein Halogenatom,

$R^3$ einen $C_1$-$C_4$-Alkylrest, einen $C_1$-$C_4$-Alkoxyrest, einen $C_1$-$C_4$-Alkylthiorest, einen $C_1$-$C_4$-Alkylaminorest, einen Di-$C_1$-$C_4$-alkylaminorest oder ein Halogenatom,

$R^4$ ein Wasserstoffatom, einen $C_1$-$C_4$-Alkylrest, einen $C_1$-$C_4$-Alkoxyrest, einen Aminorest, einen Di-$C_1$-$C_4$-alkylaminorest, einen Nitrorest, ein Halogenatom, einen Trifluormethylrest oder einen Phenylrest,

$R^5$ ein Wasserstoffatom, einen $C_1$-$C_4$-Alkylrest, einen $C_1$-$C_4$-Alkoxyrest, einen Aminorest, einen Di-$C_1$-$C_4$-alkylaminorest, einen Nitrorest, ein Halogenatom, einen Trifluormethylrest oder einen Phenylrest,

$R^6$ ein Wasserstoffatom, einen $C_1$-$C_4$-Alkylrest, einen $C_1$-$C_4$-Alkoxyrest, einen Aminorest, einen Di-$C_1$-$C_4$-alkylaminorest, einen Nitrorest, ein Halogenatom, einen Trifluormethylrest oder einen Phenylrest,

$R^7$ ein Wasserstoffatom, einen $C_1$-$C_4$-Alkylrest, einen $C_1$-$C_4$-Alkoxyrest, einen Aminorest, einen Di-$C_1$-$C_4$-alkylaminorest, einen Nitrorest, ein Halogenatom, einen Trifluormethylrest oder einen Phenylrest,

$R^8$ ein Wasserstoffatom, einen $C_1$-$C_4$-Alkylrest, einen $C_1$-$C_4$-Alkoxyrest, einen Aminorest, einen Di-$C_1$-$C_4$-alkylaminorest, einen Nitrorest, ein Halogenatom, einen Trifluormethylrest oder einen Phenylrest,

$R^9$ ein Wasserstoffatom, einen $C_1$-$C_4$-Alkylrest oder einen Phenylrest,

X ein Sauerstoffatom oder ein Schwefelatom und

Y eine Methingruppe oder ein Stickstoffatom

bedeuten, sowie deren Alkalimetallsalze, Erdalkalimetallsalze und organiischen Ammoniumsalze und deren E- und Z-Isomere.

2. Verfahren zur Herstellung von Verbindungen der allgemeinen Formel I, dadurch gekennzeichnet, daß

man

A) Verbindungen der allgemeinen Formel II

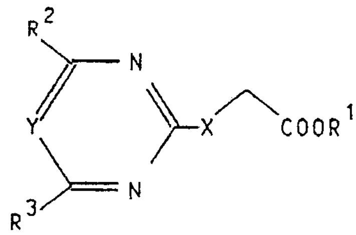

(II) ,

in der $R^1$, $R^2$, $R^3$, X und Y die unter der allgemeinen Formel I genannten Bedeutungen haben, mit Verbindungen der allgemeinen Formel III

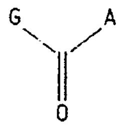

(III) ,

in der A und G die unter der allgemeinen Formel I genannten Bedeutungen haben, in einem geeigneten Lösungsmittel in Gegenwart einer geeigneten Base umsetzt, oder

B) Verbindungen der allgemeinen Formel IV

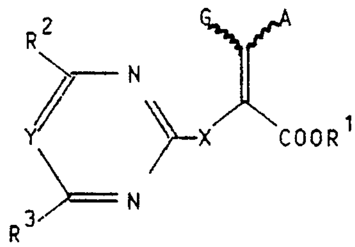

(IV) ,

in der A, G, $R^1$, $R^2$, $R^3$, X und Y die unter der allgemeinen Formel I angegebenen Bedeutungen haben, in einem geeigneten polaren Lösungsmittel mit einer Alkalimetallbase, Erdalkalimetallbase oder organischen Ammoniumbase zu Verbindungen der allgemeinen Formel V

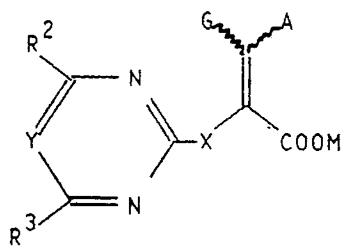

(V) ,

in der A, G, $R^2$, $R^3$, X und Y die unter der allgemeinen Formel I angegebenen Bedeutungen haben und M für ein Alkalimetallatom, ein Äquivalent eines Erdalkalimetallatoms oder eine organische Ammoniumgruppe steht, umsetzt, oder

C) Verbindungen der allgemeinen Formel V